# EUROPEAN PATENT APPLICATION

(11) **EP 1 201 240 A2**
(43) Date of publication of application: **02.05.2002**
(21) Application number: 01308061.9
(22) Date of filing: 21.09.2001
(51) Int. Cl.: A61K 31/41, A61K 31/4245, A61K 31/433, C07D 257/04, C07D 271/07, C07D 285/08, C07D 291/04, A61P 25/00, A61P 25/08, A61P 25/28, A61P 25/22, A61P 29/00, A61P 1/00, A61P 25/24

(54) **Heterocyclic derivatives useful as pharmaceutical agents**

(30) Priority: 31.10.2000 GB 0026578
(71) Applicant: WARNER-LAMBERT COMPANY, Morris Plains New Jersey 07950 (US)
(72) Inventor: Bryans, Justin Stephen, Balsham, Cambridgeshire CB1 6DZ (GB); Williams, Sophie Caroline, Cambridge, Cambridgeshire CB1 7TX (GB); Blakemore, David Clive, Cambridge, Cambridgeshire CB1 8TH (GB)
(74) Representative: Cole, Paul

(57) **Abstract**

This invention relates to novel heterocyclic derivatives of the formula (VII), (VIII) or (IX) 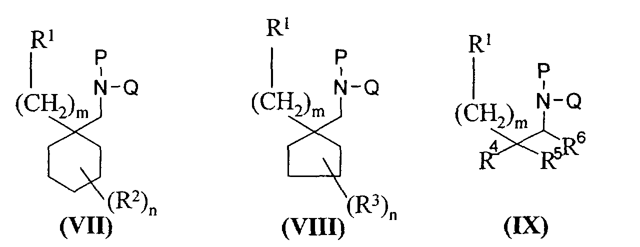 in which P, Q , R¹ - R⁶, m and n are as defined in the specification, and to pharmaceutically acceptable salts thereof. The compounds and pharmaceutical compositions containing them are useful in the treatment of a range of disorders including epilepsy, faintness attacks, hypokinesia, cranial disorders, depression, anxiety, panic, pain, neuropathological disorders, inflammatory diseases and gastrointestinal disorders, especially irritable bowel syndrome.

## Description

### FIELD OF THE INVENTION

This invention relates to novel heterocyclic derivatives useful as pharmaceutical agents, to processes for their production, to pharmaceutical compositions containing them, and to their use for the treatment of the neurological conditions set out below.

### BACKGROUND TO THE INVENTION

Gabapentin (Neurontin®) is an anti-convulsant agent that is useful in the treatment of epilepsy and that has recently been shown to be a potential treatment for neurogenic pain. It is 1-(aminomethyl)-cyclohexylacetic acid of structural formula:

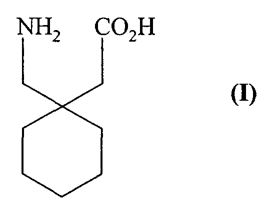

Gabapentin is one of a series of compounds of formula 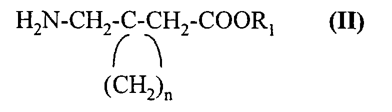 in which R₁ is hydrogen or a lower alkyl radical and n is 4, 5, or 6. These compounds are described US-A-4,024,175 and its divisional US-A-4,087,544. Their disclosed uses are: the cerebral diseases, epilepsy, faintness attacks, hypokinesia, and cranial traumas; and improvement in cerebral functions. The compounds are useful in geriatric patients. The disclosures of the above two patents are hereby incorporated by reference.

WO 97/33858 whose disclosure is incorporated herein by reference describes novel substituted cyclic amino acids, their derivatives, prodrugs and pharmaceutically acceptable salts that are of the formula: 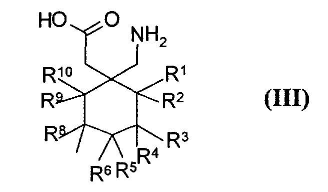 in which R¹ to R¹⁰ are each independently selected from straight or branched chain C¹ - C⁶ alkyl, substituted or unsubstituted benzyl or phenyl which substituents are selected from halogen, alkoxy, alkyl, hydroxy, carboxy, carboalkoxy, trifluoromethyl and nitro, any of R¹ to R¹⁰ which is not one of the above being hydrogen. They are useful in the treatment of epilepsy, faintness attacks, hypokinesia, cranial disorders, neurodegenerative disorders, depression, anxiety, panic, pain and neuropathological disorders.

WO 99/21824, whose disclosure is also incorporated by reference, discloses further cyclic amino acids that are useful in the treatment of epilepsy, faintness attacks, neurodegenerative disorders, depression, anxiety, panic, pain, neuropathological disorders, gastrointestinal disorders such as irritable bowel syndrome (IBS) and inflammation, especially arthritis. The compounds disclosed include those of the formula: 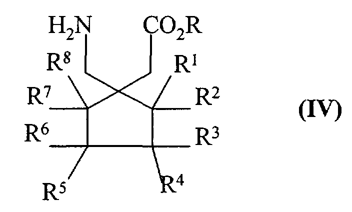 and salts thereof, in which:
R is hydrogen or a lower alkyl;
R¹ to R⁸ are each independently selected from hydrogen, straight or branched alkyl of from 1 to 6 carbons, phenyl, benzyl, fluorine, chlorine, bromine, hydroxy, hydroxymethyl, amino, aminomethyl, trifluoromethyl, -CO₂H, -CO₂R¹⁵, -CH₂CO₂H, -CH₂CO₂R¹⁵, -OR¹⁵ wherein R¹⁵ is a straight or branched alkyl of from 1 to 6 carbons, phenyl, or benzyl, and R¹ to R⁸ are not simultaneously hydrogen.

US-A-5563175 whose disclosure is incorporated herein by reference describes compounds of the formula (V) 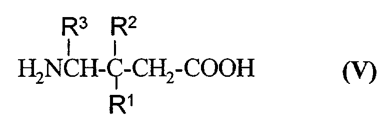 in which:
R¹ represents straight or branched C₁ - C₆ alkyl, C₃ - C₆ cycloalkyl or phenyl;
R² represents hydrogen or methyl; and
R³ represents hydrogen, methyl or carboxyl.

The compounds of formula (V) (including their pharmaceutically acceptable salts) are structural analogues of γ-aminobutyric acid (GABA) and were stated to activate L-glutamic acid decarboxylase (GAD), to bind to a novel binding site, to be useful in anti-seizure therapy for central nervous system disorders such as epilepsy, Huntington's chorea, cerebral ischemia, Parkinson's disease, tardive diskinesia and spasticity, and also to exhibit antidepressant, anxiolytic and antipsychotic activity. The most preferred compounds were those where R³ and R² were hydrogen and R¹ was isobutyl, the (S)-(+) enantiomer of formula (VI) being the most preferred.

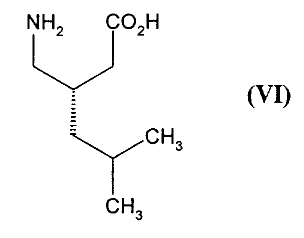

That compound is variously called 4-amino-3-(2-methylpropyl)butanoic acid, 3-(aminomethyl)-5-methylhexanoic acid, β-isobutyl-y-aminobutyric acid, isobutyl-GABA, isobutylgaba and pregabalin.

US-A-6001876 discloses that the above compounds are useful in pain therapy. US-A-5840956 discloses methods for making (±)-isobutylgaba and for obtaining from it (S)-isobutylgaba. The disclosure of these specifications is also incorporated herein by reference.

WO 99/31075 and WO 99/31074 whose disclosures are incorporated herein by reference describe *inter alia* heterocyclic analogs of the compounds of formulae (III), (IV) and (V) in which a biosterically equivalent group having acid hydrogen attached to a ring amine group replaces the carboxyl moiety. The analogs are stated to be useful as agents in the treatment of *inter alia* epilepsy, faintness attacks, hypokinesia, cranial disorders, depression, anxiety, panic, pain, neuropathological disorders, inflammatory diseases and gastrointestinal disorders, especially irritable bowel syndrome. The following compounds are disclosed as intermediates in WO 99/31075:
[1-(5-oxo-4,5-dihydro-[1,2,4]oxadiazol-3-ylmethyl)-cyclohexylmethyl]-carbamic acid tert-butyl ester; and
[1-(5-thioxo-4,5-dihydro-[1,2,4]oxadiazol-3-ylmethyl)-cyclohexylmethyl]-carbamic acid tert-butyl ester. The following compounds are disclosed as intermediates in WO 99/31074:
4-methyl-2-(1H-tetrazol-5-ylmethyl)pentyl-carbamic acid tert-butyl ester;
BOC-isobutyl GABA oxadiazolonethione; and
BOC-isobutyl GABA oxadiazolone.

### SUMMARY OF THE INVENTION

A problem with which this invention is concerned is the production of pharmaceutical compositions and active compounds useful in the manner of the heterocyclic compounds of WO 99/31075 and WO 99/31074, especially in pain therapy, that when administered to humans or other animals provide an increased duration of active ingredient in the plasma.

That problem is unexpectedly solved, according to the invention, by a pharmaceutical composition comprising a pharmaceutically acceptable carrier and a therapeutically effective amount of a compound of the formula (VII), (VIII) or (IX) or a pharmaceutically acceptable salt thereof: 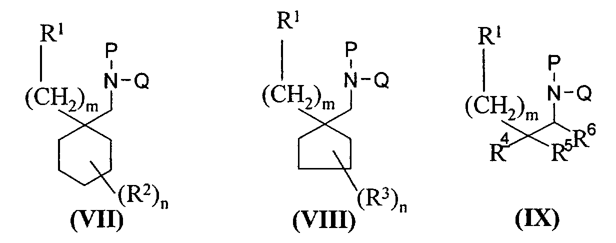 in which:
P is hydrogen or methyl;
Q is a labile amine- or amide-forming organic group that becomes removed in the human or animal, especially mammal, body;
R¹ is a heterocycle selected from: 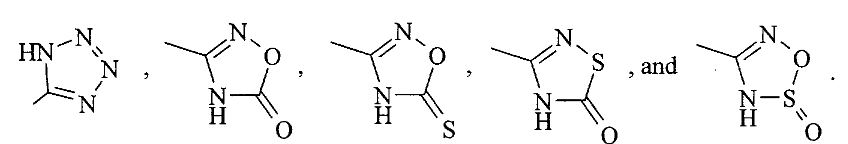
R² represents methyl; and
the groups R³ (which when n is 2 may be the same or different) represent C₁ - C₆ alkyl;
R⁴ is straight or branched C₁ - C₆ alkyl, C₃ - C₆ cycloalkyl or phenyl;
R⁵ is hydrogen or methyl;
R⁶ is hydrogen, methyl or carboxyl;
m is an integer from 0 to 2; and
n is an integer from 0 to 2.

Most of the compounds of the above formulae are new. The invention also relates to a compound of any of the formulae (VII), (VIII) or (IX) as defined in above or a pharmaceutically acceptable salt thereof, subject to the proviso that said compound is other than:
[1-(5-oxo-4,5-dihydro-[1,2,4]oxadiazol-3-ylmethyl)-cyclohexylmethyl]-carbamic acid tert-butyl ester;
[1-(5-thioxo-4,5-dihydro-[1,2,4]oxadiazol-3-ylmethyl)-cyclohexylmethyl]-carbamic acid tert-butyl ester;
4-methyl-2-(1H-tetrazol-5-ylmethyl)pentyl-carbamic acid tert-butyl ester;
BOC-isobutyl GABA oxadiazolonethione; and
BOC-isobutyl GABA oxadiazolone.

It is believed that a pro-drug of the formula (VII), (VIII) or (IX) when administered to a human or other animal, especially a mammal, enters the bloodstream by passive diffusion along the whole length of the intestine, which gives a much longer duration of effectiveness. The pro-drug may not itself be biologically active, but decomposes to the corresponding active compound in plasma.

Certain of the compounds of formula (VII), (VIII) or (IX) can exist in unsolvated forms as well as solvated forms, including hydrated forms. In general, the solvated forms, including hydrated forms, are biologically equivalent to unsolvated forms and are encompassed within the scope of the invention. Certain of the compounds of the invention possess one or more chiral centers and each center may exist in the R or S configuration. The invention includes all enantiomeric and epimeric forms as well as the appropriate mixtures thereof. It also includes salts of any of the above compounds with physiologically acceptable cations or anions.

The invention also provides a method for making a compound of the formula (VII), (VIII) or (IX) above, which comprises:
coupling a compound of the formula: 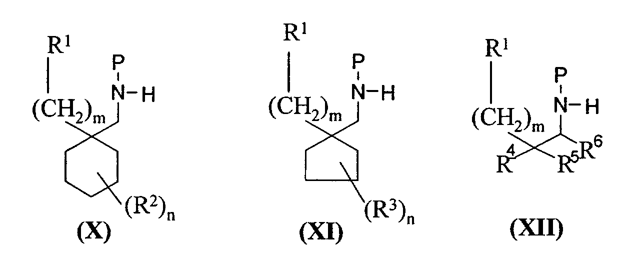 in which P and R¹ - R⁶ have the meanings given above and in which said compound is in the form of a free base or an ammonium salt with a compound of the formula (XIII) 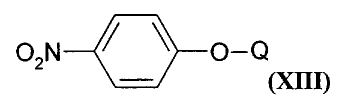
or QCl, where (in each case) Q has the meaning given above.

The invention also provides a pharmaceutical composition comprising a therapeutically effective amount of a compound of formula (VII), (VIII) or (IX) above and a pharmaceutically acceptable carrier.

In a further aspect the invention provides the use of a compound of formula (VII), (VIII) or (IX) in the manufacture of a medicament for the treatment of any of the following:
epilepsy; a faintness attack;
hypokinesia; a cranial disorder;
a neurodegenerative disorder; depression;
anxiety; panic;
pain; a neuropathological disorder;
a digestive disorder.

In a further aspect, the invention provides a method for treating any of the above disorders which comprises administering a therapeutically effective amount of a compound of formula (VII), (VIII) or (IX) to a human or animal in need of said treatment.

### DESCRIPTION OF PREFERRED FEATURES

### Preferred values for Q

The group Q may be one that can be removed hydrolytically under physiological conditions, in which case it may be 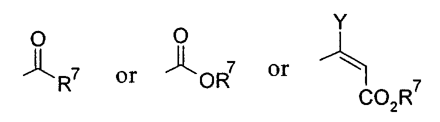 in which:
R⁷ is hydrogen, straight or branched chain C₁ - C₆ alkyl, phenyl or benzyl in which the benzene ring may be substituted or unsubstituted; and
Y is hydrogen, straight or branched chain C₁ - C₆ alkyl, or -CH₂CO₂R⁸ in which R⁸ represents straight or branched chain C₁ - C₆ alkyl

Alternatively, the group Q may be one which can be removed enzymatically under physiological conditions, in which case it may be selected from 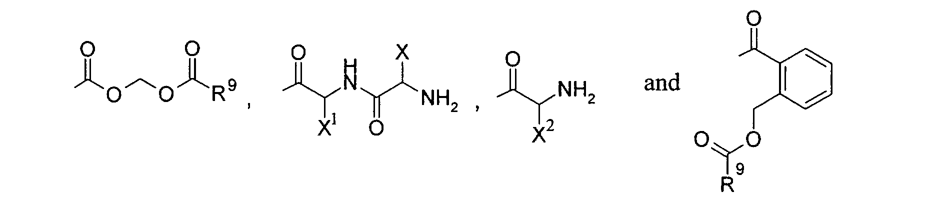 in which:
R⁹ is hydrogen, straight or branched chain, phenyl or benzyl in which either or each benzene ring may be substituted or unsubstituted; and
X, X¹ and X² represent a phenyl group or any of the side chains of the 20 naturally encoded α-amino acids.

In a preferred group of compounds Q is 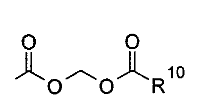 wherein R¹⁰ is C₁ - C₆ alkyl (preferably methyl or t-butyl) or phenyl.

### Active compounds providing the basis of pro-drugs

Pro-drugs according to the invention and of formulae (VII) and (VIII) may be produced corresponding to the following compounds disclosed in WO 99/31075, the -NH₂ group being replaced by a -NPQ group, where P and Q have the meanings given above:

### Tetrazoles

C-[1-(1H-Tetrazol-5-ylmethyl)-cyclohexyl]-methylamine;
(1S-cis)C-[3-Methyl-1-(1H-tetrazol-5-ylmethyl)-cyclohexyl]methylamine;
C-[1-(1H-Tetrazol-5-ylmethyl)-cyclopentyl]-methylamine;
(trans)C-[3,4-Dimethyl-1-(1H-tetrazol-5-ylmethyl)-cyclopentyl]-methylamine;
(1S-cis)C-[3-Methyl-1-(1H-tetrazol-5-ylmethyl)-cyclopentyl]-methylamine;
(1R-trans)C-[3-Methyl-1-(1H-tetrazol-5-ylmethyl)-cyclopentyl]-methylamine;
(1R-cis)C-[3-Methyl-1-(1H-tetrazol-5-ylmethyl)-cyclopentyl]-methylamine;
(1S-trans)C-[3-Methyl-1-(1H-tetrazol-5-ylmethyl)-cyclopentyl]-methylamine;
(1α,3α,4α)C-[3,4-Dimethyl-1-(1H-tetrazol-5-ylmethyl)-cyclopentyl]-methylamine;
(1α,3β,4β)C-[3,4-Dimethyl-1-(1H-tetrazol-5-ylmethyl)-cyclopentyl]-methylamine;
(S)C-[3,3-Dimethyl-1-(1 H-tetrazol-5-ylmethyl)-cyclopentyl]-methylamine;
(R)C-[3,3-Dimethyl-1-(1H-tetrazol-5-ylmethyl)-cyclopentyl]-methylamine.

### Oxadiazolones

3-(1-Aminomethyl-cyclohexylmethyl)-4H-[1,2,4]oxadiazol-5-one;
(1S-cis)3-(1-Aminomethyl-3-methyl-cyclohexylmethyl)-4H-[1,2,4]-oxadiazol-5-one;
3-(1-Aminomethyl-cyclopentylmethyl)-4H-[1,2,4]oxadiazol-5-one;
(trans)3-(1-Aminomethyl-3,4-dimethyl-cyclopentylmethyl)-4H-[1,2,4]oxadiazol-5-one;
(1S-cis)3-(1-Aminomethyl-3-methyl-cyclopentylmethyl)-4H-[1,2,4]oxadiazol-5-one;
(1R-trans)3-(1-Aminomethyl-3-methyl-cyclopentylmethyl)-4H-[1,2,4]oxadiazol-5-one;
(1R-cis)3-(1-Aminomethyl-3-methyl-cyclopentylmethyl)-4H-[1,2,4]oxadiazol-5-one;
(1S-trans)3-(1-Aminomethyl-3-methyl-cyclopentylmethyl)-4H-[1,2,4]oxadiazol-5-one;
(1α,3α,4α)3 -(1-Aminomethyl-3,4-dimethyl-cyclopentylmethyl)-4H-[ 1,2,4]oxadiazol-5-one;
(1α,3β,4β)3-(1-Aminomethyl-3,4-dimethyl-cyclopentylmethyl)-4H-[1,2,4]oxadiazol-5-one;
(S)3 -(1 -Aminomethyl-3,3 -dimethyl-cyclopentylmethyl)-4H-[1,2,4]oxadiazol-5-one;
(R)3-(1-Aminomethyl-3,3-dimethyl-cyclopentylmethyl)-4H-[1,2,4]oxadiazol-5-one.

### [1,2,4]Oxadiazole-5-thiones

3-(1-Aminomethyl-cyclohexylmethyl)-4H-[1,2,4]oxadiazol-5-thione;
(1 S-cis)3-(1-Aminomethyl-3-methyl-cyclohexylmethyl)-4H-[1,2,4]oxadiazol-5-thione;
3-(1-Aminomethyl-cyclopentylmethyl)-4H-[1,2,4]oxadiazol-5-thione;
(trans)3 -(1-Aminomethyl-3,4-dimethyl-cyclopentylmethyl)-4H-[ 1,2,4]oxadiazol-5-thione;
(1S-cis)3-(1-Aminomethyl-3-methyl-cyclopentylmethyl)-4H-[1,2,4]oxadiazol-5-thione;
(1R-trans)3-(1 -Aminomethyl-3 -methyl-cyclopentylmethyl)-4H-[1,2,4]oxadiazol-5-thione;
(1R-cis)3-(1-Aminomethyl-3-methyl-cyclopentylmethyl)-4H-[1,2,4]oxadiazol-5-thione;
(1S-trans)3-(1-Aminomethyl-3-methyl-cyclopentylmethyl)-4H-[1,2,4]oxadiazol-5-thione;
(1α,3α,4α)3-(1-Aminomethyl-3,4-dimethyl-cyclopentylmethyl)-4H-[1,2,4]oxadiazol-5-thione;
(1α,3β,4β)3-(1-Aminomethyl-3,4-dimethyl-cyclopentylmethyl)-4H-[1,2,4]oxadiazol-5-thione;
(S)3-(1-Aminomethyl-3,3-dimethyl-cyclopentylmethyl)-4H-[1,2,4]oxadiazol-5-thione;
(R)3-(1-Aminomethyl-3,3-dimethyl-cyclopentylmethyl)-4H-[1,2,4]oxadiazol-5-thione;
3-(1-Aminomethyl-3,3-dimethyl-cyclobutylmethyl)-4H-[1,2,4]oxadiazol-5-thione.

### [1,2,4]Thiadiazol-5-ones

3-(1-Aminomethyl-cyclohexylmethyl)-4H- 1,2,4]thiadiazol-5-one;
(1S-cis)3-(1 -Aminomethyl-3 -methyl-cyclohexylmethyl)-4H-[1,2,4]thiadiazol-5-one;
3-(1-Aminomethyl-cyclopentylmethyl)-4H-[1,2,4]thiadiazol-5-one;
(trans)3-(1-Aminomethyl-3,4-dimethyl-cyclopentylmethyl)-4H-[1,2,4]thiadiazol-5-one;
(1R-cis)3-(1-Aminomethyl-3-methyl-cyclopentylmethyl)-4H-[ 1,2,4]thiadiazol-5-one;
(1S-trans)3-(1-Aminomethyl-3-methyl-cyclopentylmethyl)-4H-[1,2,4]thiadiazol-5-one;
(1S-cis)3-(1-Aminomethyl-3-methyl-cyclopentylmethyl)-4H-[1,2,4]thiadiazol-5-one;
(1R-trans)3-(1-Aminomethyl-3 -methyl-cyclopentylmethyl)-4H-[1,2,4]thiadiazol-5-one;
(1α,3α,4α)3 -(1-Aminomethyl-3,4-dimethyl-cyclopentylmethyl)-4H-[1,2,4]thiadiazol-5-one;
(1α,3β,4β)3-(1-Aminomethyl-3,4-dimethyl-cyclopentylmethyl)-4H-[1,2,4]thiadiazol-5-one;
(S)3-(1-Aminomethyl-3,3-dimethyl-cyclopentylmethyl)-4H-[1,2,4]thiadiazol-5-one;
(R)3-(1-Aminomethyl-3,3-dimethyl-cyclopentylmethyl)-4H-[1,2,4]thiadiazol-5-one.

### Oxathiadiazoles

C-[1-(2-Oxo-2,3-dihydro-2λ⁴-[1,2,3,5]oxathiadiazol-4-ylmethyl)-cyclohexyl]-methylamine;
(1S-cis)C-[3-Methyl-1-(2-oxo-2,3-dihydro-2λ⁴-[1,2,3,5]oxathiadiazol-4-ylmethyl)-cyclohexyl]-methylamine;
C-[1-(2-Oxo-2,3-dihydro-2λ⁴-[1,2,3,5]oxathiadiazol-4-ylmethyl)-cyclopentyl]-methylamine;
(trans)C-[3,4-Dimethyl-1-(2-oxo-2,3-dihydro-2λ⁴-[1,2,3,5]oxathiadiazol-4-ylmethyl)-cyclopentyl]-methylamine;
(1S-cis)C-[3-Methyl-1-(2-oxo-2,3-dihydro-2λ⁴-[1,2,3,5]oxathiadiazol-4-ylmethyl)-cyclopentyl]-methylamine;
(1R-trans)C-[3-Methyl-1-(2-oxo-2,3-dihydro-2λ⁴-[1,2,3,5]oxathiadiazol-4-ylmethyl)-cyclopentyl]-methylamine;
(1R-cis)C-[3-Methyl-1-(2-oxo-2,3-dihydro-2λ⁴-[1,2,3,5]oxathiadiazol-4-ylmethyl)-cyclopentyl]-methylamine;
(1S-trans)C-[3-Methyl-1-(2-oxo-2,3-dihydro-2λ⁴-[1,2,3,5]oxathiadiazol-4-ylmethyl)-cyclopentyl]-methylamine;
(1α,3α,4α)C-[3,4-Dimethyl-1-(2-oxo-2,3-dihydro-2λ⁴-[1,2,3,5]oxathiadiazol-4-ylmethyl)-cyclopentyl]-methylamine;
(1α,3β,4β)C-[3,4-Dimethyl-1-(2-oxo-2,3-dihydro-2λ⁴-[1,2,3,5]oxathiadiazol-4-ylmethyl)-cyclopentyl]-methylamine;
(S)C-[3,3-Dimethyl-1-(2-oxo-2,3-dihydro-2λ⁴-[1,2,3,5]oxathiadiazol-4-ylmethyl)-cyclopentyl]-methylamine;
(R)C-[3,3-Dimethyl-1-(2-oxo-2,3-dihydro-2λ⁴-[1,2,3,5]oxathiadiazol-4-ylmethyl)-cyclopentyl]-methylamine.

Of the above compounds, C-[1-(1H-tetrazol-5-ylmethyl)cyclohexyl]-methylamine and 4-methyl-2-(1H-tetrazol-5-ylmethyl)-pentylamine) are at present preferred.

### Compounds of formula (IX)

Pro-drugs of formula (IX) may be made corresponding e.g. to any of the following compounds disclosed in WO 99/31074:
4-methyl-2-(1H-tetrazol-5-ylmethyl)-pentylamine;
3-(2-aminomethyl-4-methylpentyl)-4H-[1,2,4]oxadiazol-5-one, HCl;
3-(2-aminomethyl-4-methylpentyl)-4H-[1,2,4]oxadiazol-5-thione, HCl;
3-(3-amino-2-cyclopentyl-propyl)-4H-[1,2,4]oxadiazol-5-one.
2-cyclopentyl-3-(2-oxo-2,3-dihydro-2λ⁴-[1,2,3,5]oxathiadiazol-4-yl propylamine.

### Preparative methods

Various methods may be used to prepare compounds according to the invention from starting materials of formulae (X), (XI) or (XII). For example, an amide prodrug of any the above starting materials may be prepared by reacting the starting material with an acid chloride in an ether e.g. tetrahydrofuran at ambient temperatures. An (acyloxy)alkyl carbamate prodrug of the above starting materials may be prepared by reacting the starting material with an acyloxyalkyl *p*-nitrophenyl carbonate in an ether e.g. tetrahydrofuran at ambient temperatures.

### Use of the compounds

The compounds of the invention are expected to be useful in the treatment of epilepsy. They may also be used as mimetic agents for neurodegenerative disorders. Such neurodegenerative disorders are, for example, Alzheimer's disease, Huntington's disease, Parkinson's disease, and Amyotrophic Lateral Sclerosis. The present invention also covers treating acute brain injuries. These include but are not limited to: stroke, head trauma, and asphyxia. Stroke refers to a cerebral vascular disease and may also be referred to as a cerebral vascular incident (CVA) and includes acute thromboembolic stroke. Stroke includes both focal and global ischemia. Also, included are transient cerebral ischemic attacks and other cerebral vascular problems accompanied by cerebral ischemia such as in a patient undergoing carotid endarterectomy specifically or other cerebrovascular or vascular surgical procedures in general, or diagnostic vascular procedures including cerebral angiography and the like. Other incidents are head trauma, spinal cord trauma, or injury from general anoxia, hypoxia, hypoglycemia, hypotension as well as similar injuries seen during procedures from embole, hyperfusion, and hypoxia. Treatment with the present compounds could also be useful in a range of incidents, for example, during cardiac bypass surgery, in incidents of intracranial hemorrhage, in perinatal asphyxia, in cardiac arrest, and status epilepticus. A skilled physician will be able to determine the appropriate situation in which subjects are susceptible to or at risk of, for example, stroke as well as suffering from stroke for administration by methods of the present invention.

The compounds of the invention are also expected to be useful in the treatment of depression. Depression can be the result of organic disease, secondary to stress associated with personal loss, or idiopathic in origin. There is a strong tendency for familial occurrence of some forms of depression suggesting a mechanistic cause for at least some forms of depression. The diagnosis of depression is made primarily by quantification of alterations in patients' mood. These evaluations of mood are generally performed by a physician or quantified by a neuropsychologist using validated rating scales, such as the Hamilton Depression Rating Scale or the Brief Psychiatric Rating Scale. Numerous other scales have been developed to quantify and measure the degree of mood alterations in patients with depression, such as insomnia, difficulty with concentration, lack of energy, feelings of worthlessness, and guilt. The standards for diagnosis of depression as well as all psychiatric diagnoses are collected in the Diagnostic and Statistical Manual of Mental Disorders (Fourth Edition) referred to as the DSM-IV-R manual published by the American Psychiatric Association, 1994.

The present compounds are also expected to be useful in the treatment of anxiety and of panic as demonstrated by means of standard pharmacological procedures.

The compounds of the invention are also expected to be useful in the treatment of pain. Pain refers to acute as well as chronic pain. Acute pain is usually short-lived and is associated with hyperactivity of the sympathetic nervous system. Examples are postoperative pain and allodynia. Chronic pain is usually defined as pain persisting from 3 to 6 months and includes somatogenic pains and psychogenic pains. Other pain is nociceptive. Still other pain is caused by injury or inflammation of peripheral sensory nerves. It includes, but is not limited to pain from peripheral nerve trauma, herpes virus infection, diabetes mellitus, causalgia, plexus avulsion, neuroma, limb amputation, and vasculitis. Neuropathic pain is also caused by nerve damage from chronic alcoholism, human immunodeficiency virus infection, hypothyroidism, uremia, or vitamin deficiencies. Neuropathic pain includes, but is not limited to pain caused by nerve injury such as, for example, the pain diabetics suffer from. Psychogenic pain is that which occurs without an organic origin such as low back pain, atypical facial pain, and chronic headache. Other types of pain are: inflammatory pain, osteoarthritic pain, trigeminal neuralgia, cancer pain, diabetic neuropathy, restless leg syndrome, acute herpetic and postherpetic neuralgia, causalgia, brachial plexus avulsion, occipital neuralgia, gout, phantom limb, burn, and other forms of neuralgia, neuropathic and idiopathic pain syndrome.

The present compounds are also expected to be useful in the treatment of digestive disorders such as visceral pain, pain associated with cancer, the irritable bowel syndrome, infection and inflammation.

### Dosage forms

The present compounds can be prepared and administered in a wide variety of oral and parenteral dosage forms. Thus, they can be administered by injection, that is, intravenously, intramuscularly, intracutaneously, subcutaneously, intraduodenally, or intraperitoneally. Also, they can be administered by inhalation, for example, intranasally. Additionally, the compounds of the present invention can be administered transdermally. It will be obvious to those skilled in the art that the following dosage forms may comprise as the active component either a compound of the invention or a corresponding pharmaceutically acceptable salt. Oral dosage forms are preferred, but parenteral dosage forms may also be used where it is desired to use the kinetics of decomposition into the corresponding active compound.

For preparing pharmaceutical compositions from the present compounds, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid that is in a mixture with the finely divided active component. In tablets, the active component is mixed with the carrier having the necessary binding properties in suitable proportions and compacted in the shape and size desired. The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted, and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 1 g according to the particular application and the potency of the active component. In medical use the drug may be administered three times daily as, for example, capsules of 100 or 300 mg. The composition can, if desired, also contain other compatible therapeutic agents.

In therapeutic use, the compounds utilized in the pharmaceutical method of this invention are administered at the initial dosage of about 0.01 mg to about 100 mg/kg daily. A daily dose range of about 0.01 mg to about 100 mg/kg is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages that are less than the optimum dose of the compound. Thereafter, the dosage is increased by small increments until the optimum effect under the circumstances is reached. For convenience, the total daily dosage may be divided and administered in portions during the day, if desired.

### PREPARATION OF REAGENTS

Preparation of reagents for making pro-drug according to the invention is set out below.

### Acetoxymethylp-nitrophenyl carbonate (1)

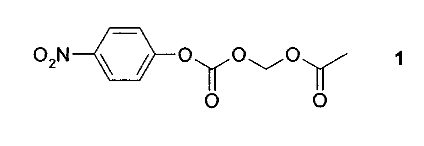

Carbonate 1 was prepared as described in *J.Med.Chem*, 1988, **31**, 318-322 (5.29 g, 98%). Its characteristics were described in *J.Org.Chem*, 1997, **62**, 1356-1362.
νₘₐₓ(film)/cm⁻¹ 1776 (C=O), 1526 (C=C, Ar).
δ_{H}(400 MHz; CDCl₃) 2.19 (3H, s, C*H*₃), 5.88 (2H, s, OC*H*₂O), 7.42 (2H, d, *J* 9.6, *p*-NO₂Ar*H*), 8.30 (2H, d, *J* 9.2, *p*-NO₂Ar*H*).

### 2,2-dimethylpropionyloxymethyl p-nitrophenyl carbonate (2)

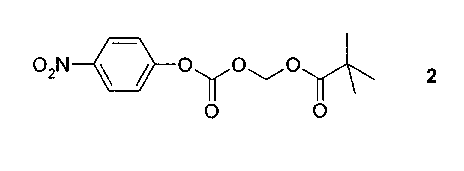

Carbonate **2** was also prepared as described in the above paper (1.16 g, 60%).
νₘₐₓ(film)/cm⁻¹ 1779, 1759 (C=O), 1530 (C=C, Ar).
δ_{H}(400 MHz; CDCl₃) 1.26 (9H, s, ^{t}butyl), 5.89 (2H, s, OC*H*₂O), 7.41 (2H, d, *J* 9.4, *p*-NO₂Ar*H*), 8.30 (2H, d, *J* 9.2, *p*-NO₂Ar*H*).

### Benzoyloxymethyl p-nitrophenyl carbonate (3)

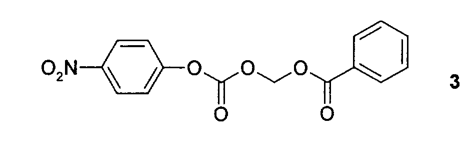

Carbonate **3** was also prepared as described in the above paper (1.76 g, 85%).
νₘₐₓ(film)/cm⁻¹ 1778, 1740 (C=O), 1525 (C=C Ar).
δ_{H}(400 MHz; CDCl₃) 6.14 (2H, s, OC*H*₂O), 7.42 (2H, d, *J* 9.2, *p*-NO₂Ar*H*), 7.49 (2H, t, *J* 8.0, Ar*H*), 7.64 (1H, t, *J* 7.6, Ar*H*), 8.12 (2H, d, *J* 7.2, Ar*H*) 8.29 (2H, d, *J* 9.2, *p*-NO₂Ar*H*).

The invention will now be further described with reference to the following examples:

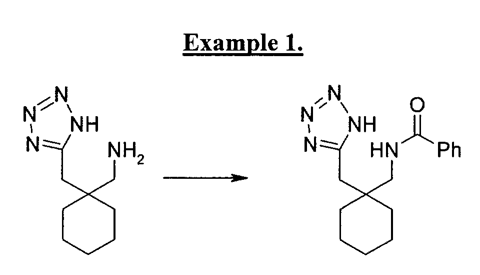

The starting 1-(1H-tetrazol-5-ylmethyl)-cycohexanecarbonitrile was synthesized as described in WO 9931075. The tetrazole (200mg, 1.026mmol) was suspended in dry dichloromethane (10ml) and stirred under nitrogen with triethylamine (340µl, 2.58mmol) and benzoyl chloride (133mg, 0.95mmol). After 24 hours, the mixture was diluted with dichloromethane (20ml) and washed with 2N HCl (aq) (20ml). The organic phase was collected, dried (MgSO₄) and the solvent removed *in vacuo* to give 225mg (73%) of the desired product as a white solid.

¹H NMR (CDCl₃, 400MHz) δ: 1.17-1.80 (10H, m), 2.97 (2H, s), 3.34 (2H, d, J=7Hz), 6.74 (1H, br. S), 7.44-7.61 (3H, m), 7.84 (2H, m).

MS (AP⁺) m/e: 300 (MH⁺, 100%).

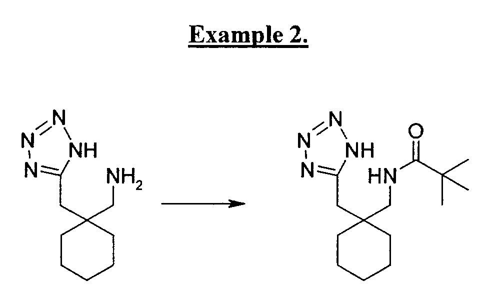

The above tetrazole (200mg, 1.026mmol) was suspended in dry dichloromethane (10ml) and stirred under nitrogen with triethylamine (340µl, 2.58mmol) and trimethylacetyl chloride (115mg, 0.95mmol). After 24 hours, the mixture was diluted with dichloromethane (20ml) and washed with 2N HCl (aq) (20ml). The organic phase was collected, dried (MgSO₄) and the solvent removed *in vacuo* to give 211 mg (74%) of the desired product as a white solid.

¹H NMR (CDCl₃, 400MHz) δ: 1.06-1.79 (10H, m), 1.28 (9H, s), 2.83 (2H, s), 3.11 (2H, d, J=7Hz), 6.10 (1H, br. S).

MS (AP⁺) m/e: 280 (MH⁺, 100%).

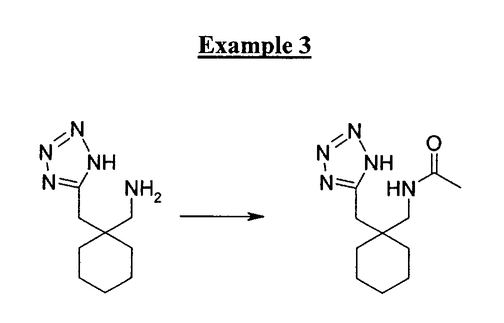

The above tetrazole (200mg, 1.026mmol) was suspended in dry dichloromethane (10ml) and stirred under nitrogen with triethylamine (340µl, 2.58mmol) and acetyl chloride (75mg, 0.95mmol). After 24 hours, the mixture was diluted with dichloromethane (20ml) and washed with 2N HCl (aq) (20ml). The organic phase was collected, dried (MgSO₄) and the solvent removed *in vacuo* to give 129mg (51%) of the desired product as a white solid.

¹H NMR (CDCl₃, 400MHz) δ: 1.08-1.77 (10H, m), 2.16 (3H, s), 2.90 (2H, s), 3.12 (2H, d, J=7Hz), 6.07 (1H, br. S).

MS (AP⁺) m/e: 238 (MH⁺, 100%).

## Claims

1. A pharmaceutical composition comprising a pharmaceutically acceptable carrier and a therapeutically effective amount of a compound of the formula (VII), (VIII) or (IX) or a pharmaceutically acceptable salt thereof: 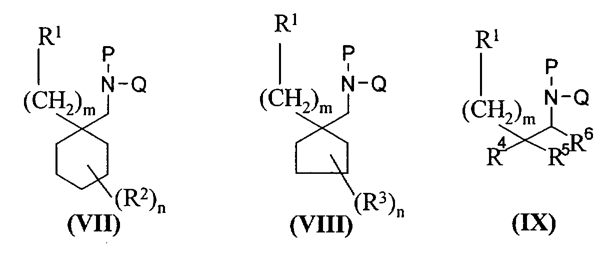 in which:
P is hydrogen or methyl;
Q is a labile amine- or amide-forming organic group that becomes removed in the human or animal, especially mammal, body;
R¹ is a heterocycle selected from: 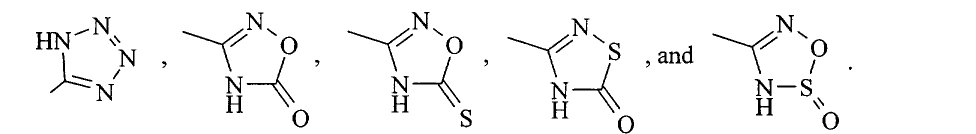
R² represents methyl;
the groups R³ (which n is 2 may be the same or different) represent C₁ - C₆ alkyl;
R⁴ is straight or branched C₁ - C₆ alkyl, C₃ - C₆ cycloalkyl or phenyl;
R⁵ is hydrogen or methyl;
R⁶ is hydrogen, methyl or carboxyl;
m is an integer from 0 to 2; and
n is an integer from 0 to 2.

2. The composition of claim 1, wherein P in the compound of the formula (VII), (VIII) or (IX) is hydrogen.

3. The composition of claim 1 or 2, wherein Q in the compound of the formula (VII), (VIII) or (IX) can be removed hydrolytically under physiological conditions.

4. The composition of claim 3, wherein Q in the compound of the formula (VII), (VIII) or (IX) is 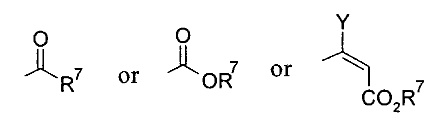 in which:
R⁷ is hydrogen, straight or branched chain C₁ - C₆ alkyl, phenyl or benzyl in which the benzene ring may be substituted or unsubstituted; and
Y is hydrogen, straight or branched chain C₁ - C₆ alkyl, or -CH₂CO₂R⁸ in which R⁸ represents straight or branched chain C₁ - C₆ alkyl.

5. The composition of claim 1 or 2, wherein Q in the compound of the formula (VII), (VIII) or (IX) can be removed enzymatically under physiological conditions.

6. The composition of claim 5, wherein the group Q in the compound of the formula (VII), (VIII) or (IX) is selected from 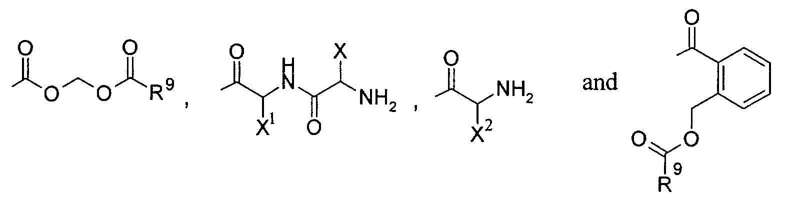 in which:
R⁹ is hydrogen, straight or branched chain, phenyl or benzyl in which either or each benzene ring may be substituted or unsubstituted; and
X, X¹ and X² represent a phenyl group or any of the side chains of the 20 naturally encoded α-amino acids.

7. The composition of claim 6, wherein Q in the compound of the formula (VII), (VIII) or (IX) is 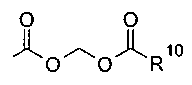 wherein R¹⁰ is C₁ - C₆ alkyl (preferably methyl or t-butyl) or phenyl.

8. The composition of claim 7, wherein R¹⁰ in the above compound is methyl or *t*-butyl.

9. The composition of any preceding claim, wherein the compound of the formula (VII), (VIII) or (IX) is a tetrazole pro-drug in which the -NH₂ group of any compound listed below is replaced by a -NPQ group, where P and Q have the meanings given above:
C-[1-(1H-Tetrazol-5-ylmethyl)-cyclohexyl]-methylamine;
(1S-cis)C-[3-Methyl-1-(1H-tetrazol-5-ylmethyl)-cyclohexyl]methylamine;
C-[1-(1H-Tetrazol-5-ylmethyl)-cyclopentyl]-methylamine;
(trans)C-[3,4-Dimethyl-1-(1H-tetrazol-5-ylmethyl)-cyclopentyl]-methylamine;
(1S-cis)C-[3-Methyl-1-(1H-tetrazol-5-ylmethyl)-cyclopentyl]-methylamine;
(1R-trans)C-[3-Methyl-1-(1H-tetrazol-5 -ylmethyl)-cyclopentyl]-methylamine;
(1R-cis)C-[3-Methyl-1-(1H-tetrazol-5-ylmethyl)-cyclopentyl]-methylamine;
(1S-trans)C-[3-Methyl-1-(1H-tetrazol-5-ylmethyl)-cyclopentyl]-methylamine;
(1α,3α,4α)C-[3,4-Dimethyl-1-(1H-tetrazol-5-ylmethyl)-cyclopentyl]-methylamine;
(1α,3β,4β)C-[3,4-Dimethyl-1-(1H-tetrazol-5-ylmethyl)-cyclopentyl]-methylamine;
(S)C-[3,3-Dimethyl-1-(1H-tetrazol-5-ylmethyl)-cyclopentyl]-methylamine;
(R)C-[3,3-Dimethyl-1-(1H-tetrazol-5-ylmethyl)-cyclopentyl]-methylamine.

10. The composition of any of claims 1-8, wherein the compound of the formula (VII), (VIII) or (IX) is an oxadiazolone pro-drug in which the -NH₂ group of any compound listed below is replaced by a -NPQ group, where P and Q have the meanings given above:
3-(1-Aminomethyl-cyclohexylmethyl)-4H-[1,2,4]oxadiazol-5-one;
(1S-cis)3-(1-Aminomethyl-3-methyl-cyclohexylmethyl)-4H-[1,2,4]-oxadiazol-5-one;
3-(1-Aminomethyl-cyclopentylmethyl)-4H-[1,2,4]oxadiazol-5-one;
(trans)3 -(1-Aminomethyl-3,4-dimethyl-cyclopentylmethyl)-4H-[1,2,4]oxadiazol-5-one;
(1S-cis)3-(1-Aminomethyl-3-methyl-cyclopentylmethyl)-4H-[1,2,4]oxadiazol-5-one;
(1R-trans)3-(1-Aminomethyl-3-methyl-cyclopentylmethyl)-4H-[1,2,4]oxadiazol-5-one;
(1R-cis)3-(1-Aminomethyl-3 -methyl-cyclopentylmethyl)-4H-[1,2,4]oxadiazol-5-one;
(1S-trans)3-(1-Aminomethyl-3 -methyl-cyclopentylmethyl)-4H-[1,2,4]oxadiazol-5-one;
(1α,3α,4α)3-(1 -Aminomethyl-3,4-dimethyl-cyclopentylmethyl)-4H-[1,2,4]oxadiazol-5-one;
(1α,3β,4β)3-(1-Aminomethyl-3,4-dimethyl-cyclopentylmethyl)-4H-[1,2,4]oxadiazol-5-one;
(S)3-(1-Aminomethyl-3,3-dimethyl-cyclopentylmethyl)-4H-[1,2,4]oxadiazol-5-one;
(R)3-(1-Aminomethyl-3,3-dimethyl-cyclopentylmethyl)-4H-[1,2,4]oxadiazol-5-one.

11. The composition of any of claims 1-8, wherein the compound of the formula (VII), (VIII) or (IX) is a [1,2,4]oxadiazole-5-thione pro-drug in which the -NH₂ group of any compound listed below is replaced by a -NPQ group, where P and Q have the meanings given above:
3-(1-Aminomethyl-cyclohexylmethyl)-4H-[1,2,4]oxadiazol-5-thione;
(1S-cis)3-(1-Aminomethyl-3-methyl-cyclohexylmethyl)-4H-[1,2,4]oxadiazol-5-thione;
3-(1-Aminomethyl-cyclopentylmethyl)-4H-[1,2,4]oxadiazol-5-thione;
(trans)3 -(1-Aminomethyl-3,4-dimethyl-cyclopentylmethyl)-4H-[1,2,4]oxadiazol-5-thione;
(1S-cis)3-(1-Aminomethyl-3-methyl-cyclopentylmethyl)-4H-[1,2,4]oxadiazol-5-thione;
(1R-trans)3-(1-Aminomethyl-3 -methyl-cyclopentylmethyl)-4H-[1,2,4]oxadiazol-5-thione;
(1R-cis)3-(1-Aminomethyl-3-methyl-cyclopentylmethyl)-4H-[1,2,4]oxadiazol-5-thione;
(1S-trans)3-(1-Aminomethyl-3-methyl-cyclopentylmethyl)-4H-[1,2,4]oxadiazol-5-thione;
(1α,3α,4α)3-(1-Aminomethyl-3,4-dimethyl-cyclopentylmethyl)-4H-[1,2,4]oxadiazol-5-thione;
(1α,3β,4β)3-(1-Aminomethyl-3,4-dimethyl-cyclopentylmethyl)-4H-[1,2,4]oxadiazol-5-thione;
(S)3-(1-Aminomethyl-3,3-dimethyl-cyclopentylmethyl)-4H-[1,2,4]oxadiazol-5-thione;
(R)3-(1-Aminomethyl-3,3-dimethyl-cyclopentylmethyl)-4H-[1,2,4]oxadiazol-5-thione;
3-(1-Aminomethyl-3,3-dimethyl-cyclobutylmethyl)-4H-[1,2,4]oxadiazol-5-thione.

12. The composition of any of claims 1-8, wherein the compound of the formula (VII), (VIII) or (IX) is a [1,2,4]thiadiazol-5-one pro-drug in which the - NH₂ group of any compound listed below is replaced by a -NPQ group, where P and Q have the meanings given above:
3-(1-Aminomethyl-cyclohexylmethyl)-4H-[1,2,4]thiadiazol-5-one;
(1S-cis)3-(1-Aminomethyl-3-methyl-cyclohexylmethyl)-4H-[1,2,4]thiadiazol-5-one;
3-(1-Aminomethyl-cyclopentylmethyl)-4H-[1,2,4]thiadiazol-5-one;
(trans)3 -(1-Aminomethyl-3,4-dimethyl-cyclopentylmethyl)-4H-[1,2,4]thiadiazol-5-one;
(1R-cis)3-(1-Aminomethyl-3-methyl-cyclopentylmethyl)-4H-[1,2,4]thiadiazol-5-one;
(1S-trans)3-(1-Aminomethyl-3-methyl-cyclopentylmethyl)-4H-[1,2,4]thiadiazol-5-one;
(1S-cis)3-(1-Aminomethyl-3-methyl-cyclopentylmethyl)-4H-[1,2,4]thiadiazol-5-one;
(1R-trans)3-(1-Aminomethyl-3-methyl-cyclopentylmethyl)-4H-[1,2,4]thiadiazol-5-one;
(1α,3α,4α)3-(1-Aminomethyl-3,4-dimethyl-cyclopentylmethyl)-4H-[1,2,4]thiadiazol-5-one;
(1α,3β,4β)3-(1-Aminomethyl-3,4-dimethyl-cyclopentylmethyl)-4H-[1,2,4]thiadiazol-5-one;
(S)3-(1-Aminomethyl-3,3-dimethyl-cyclopentylmethyl)-4H-[1,2,4]thiadiazol-5-one;
(R)3-(1-Aminomethyl-3,3-dimethyl-cyclopentylmethyl)-4H-[1,2,4]thiadiazol-5-one.

13. The composition of any of claims 1-8, wherein the compound of the formula (VII), (VIII) or (IX) is an oxathiadiazole pro-drug in which the -NH₂ group of any compound listed below is replaced by a -NPQ group, where P and Q have the meanings given above:
C-[1-(2-Oxo-2,3-dihydro-2λ⁴-[1,2,3,5]oxathiadiazol-4-ylmethyl)-cyclohexyl]-methylamine;
(1S-cis)C-[3-Methyl-1-(2-oxo-2,3-dihydro-2λ⁴-[1,2,3,5]oxathiadiazol-4-ylmethyl)-cyclohexyl]-methylamine;
C-[1-(2-Oxo-2,3-dihydro-2λ⁴-[1,2,3,5]oxathiadiazol-4-ylmethyl)-cyclopentyl] -methylamine;
(trans)C-[3,4-Dimethyl-1-(2-oxo-2,3-dihydro-2λ⁴-[1,2,3,5]oxathiadiazol-4-ylmethyl)-cyclopentyl]-methylamine;
(1S-cis)C-[3-Methyl-1-(2-oxo-2,3-dihydro-2λ⁴-[1,2,3,5]oxathiadiazol-4-ylmethyl)-cyclopentyl]-methylamine;
(1R-trans)C-[3-Methyl-1-(2-oxo-2,3-dihydro-2λ⁴-[1,2,3,5]oxathiadiazol-4-ylmethyl)-cyclopentyl]-methylamine;
(1R-cis)C-[3-Methyl-1-(2-oxo-2,3-dihydro-2λ⁴-[1,2,3,5]oxathiadiazol-4-ylmethyl)-cyclopentyl]-methylamine;
(1S-trans)C-[3-Methyl-1-(2-oxo-2,3-dihydro-2λ⁴-[1,2,3,5]oxathiadiazol-4-ylmethyl)-cyclopentyl]-methylamine;
(1α,3α,4α)C-[3,4-Dimethyl-1-(2-oxo-2,3-dihydro-2λ⁴-[1,2,3,5]oxathiadiazol-4-ylmethyl)-cyclopentyl]-methylamine;
(1α,3β,4β)C-[3,4-Dimethyl-1-(2-oxo-2,3-dihydro-2λ⁴-[1,2,3,5]oxathiadiazol-4-ylmethyl)-cyclopentyl]-methylamine;
(S)C-[3,3-Dimethyl-1-(2-oxo-2,3-dihydro-2λ⁴-[1,2,3,5]oxathiadiazol-4-ylmethyl)-cyclopentyl]-methylamine;
(R)C-[3,3-Dimethyl-1-(2-oxo-2,3-dihydro-2λ⁴-[1,2,3,5]oxathiadiazol-4-ylmethyl)-cyclopentyl]-methylamine.

14. The composition of any of claims 1-8, wherein the compound of the formula (VII), (VIII) or (IX) is a pro-drug of formula (IX) in which the -NH₂ group of any compound listed below is replaced by a -NPQ group, where P and Q have the meanings given above:
4-methyl-2-(1H-tetrazol-5-ylmethyl)-pentlyamine;
3-(2-aminomethyl-4-methylpentyl)-4H-[1,2,4]oxadiazol-5-one, HCl;
3-(2-aminomethyl-4-methylpentyl)-4H-[1,2,4]oxadiazole-5-thione, HCl;
3-(3-amino-2-cyclopentyl-propyl)-4H-[1,2,4]oxadiazol-5-one.
2-cyclopentyl-3-(2-oxo-2,3-dihydro-2λ⁴-[1,2,3,5]oxathiadiazol-4-yl propylamine.

15. The composition of claim 1 comprising a therapeutically effective amount of the following compound or a pharmaceutically acceptable salt thereof.

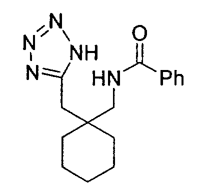

16. The composition of claim 1 comprising a therapeutically effective amount of the following compound or a pharmaceutically acceptable salt thereof:

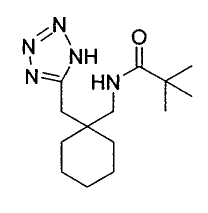

17. The composition of claim 1 comprising a therapeutically effective amount of the following compound or a pharmaceutically acceptable salt thereof:

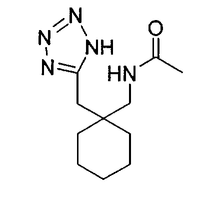

18. Use of any compound of any of the formulae (VII), (VIII) or (IX) as defined in any of claims 1-17 or a pharmaceutically acceptable salt thereof in the manufacture of a medicament for the treatment of any of the following:
epilepsy; a faintness attack;
hypokinesia; a cranial disorder;
a neurodegenerative disorder; depression;
anxiety; panic;
pain; a neuropathological disorder;
a digestive disorder.

19. A method for treating any of the disorders defined in claim 18, which comprises administering a therapeutically effective amount of a compound as defined in any of claims 1-17 to a human or animal in need of said treatment.

20. A compound of any of the formulae (VII), (VIII) or (IX) as defined in any of claims 1-17 or a pharmaceutically acceptable salt thereof, subject to the proviso that said compound is other than:
[1-(5-oxo-4,5-dihydro-[1,2,4]oxadiazol-3-ylmethyl)-cyclohexylmethyl]-carbamic acid tert-butyl ester;
[1-(5-thioxo-4,5-dihydro-[1,2,4]oxadiazol-3-ylmethyl)-cyclohexylmethyl]-carbamic acid tert-butyl ester;
4-methyl-2-(1H-tetrazol-5-ylmethyl)pentyl-carbamic acid tert-butyl ester;
BOC-isobutyl GABA oxadiazolonethione; and
BOC-isobutyl GABA oxadiazolone.

21. A method for making a compound of the formula (VII), (VIII) or (IX) as defined in any of claims 1-17, which comprises:
coupling a compound of the formula: 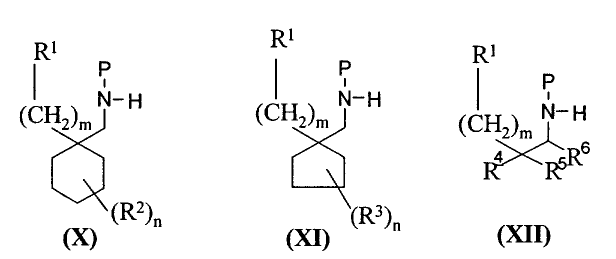 in which P and R¹ - R⁶ have the meanings given above and in which said compound is in the form of a free base or an ammonium salt with a compound of the formula (XIII) 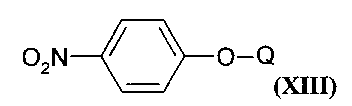
or QCl, where (in each case) Q has the meaning given above.
